# EUROPEAN PATENT SPECIFICATION

(11) **EP 3 316 911 B1**
(45) Date of publication and mention of the grant of the patent: **04.11.2020**
(21) Application number: 16733306.1
(22) Date of filing: 29.06.2016
(51) Int. Cl.: A61K 47/36, A61K 8/04, A61Q 19/08, A61Q 19/00, A61K 8/73, C08B 37/00, C08L 5/08, A61K 31/728, A61K 31/245, C08J 3/00, A61K 31/167, C08B 37/08

(54) **METHOD OF PREPARING A COMPOSITION BASED ON HYALURONIC ACID**
VERFAHREN ZUR HERSTELLUNG EINER ZUSAMMENSETZUNG ENTHALTEND HYALURONSÄURE
PROCÉDÉ DE PRÉPARATION D'UNE COMPOSITION À BASE D'ACIDE HYALURONIQUE

(30) Priority: 30.06.2015 EP 15001938
(43) Date of publication of application: 09.05.2018
(73) Proprietor: Merz Pharma GmbH & Co. KGaA, 60318 Frankfurt am Main (DE)
(72) Inventor: BELKOVI, Lubin, 61381 Friedrichsdorf (DE); VILLAIN, Franck, 75020 Paris (FR); LINKO, Alexander, 60599 Frankfurt am Main (DE); PLEIS, Frank, 60385 Frankfurt am Main (DE)
(74) Representative: Wallinger Ricker Schlotter Tostmann
(86) International application number: PCT/EP2016/001111
(87) International publication number: WO 2017/001057

(56) References cited:
- WO-A1-2013/185934
- US-A1- 2010 028 437
- US-A1- 2013 203 856

## Description

### FIELD OF THE INVENTION

The invention relates to a composition based on a polysaccharide, such as hyaluronic acid or a sodium salt thereof, having improved homogeneity and degradation stability, to a method of preparing said composition, and to the use of the composition as dermatological filler, wherein said composition may include an anesthetic and/or anti-arrhythmic such as lidocain.

### BACKGROUND OF THE INVENTION

It is known to use gels such as hydrogels based on polysaccharides and water as dermatological fillers. Such gels are generally prepared by methods comprising the chemical cross-linking of the respective polysaccharides in an aqueous medium. Suitable polysaccharides are e.g. based on hyaluronic acid since it is present in identical or similar compositions in each living organism. E.g., hyaluronic acid (HA) is a major component of skin, where it is involved in tissue repair. Therefore, it gives a minimum of side effects and allows for safe application.

EP 1 818 344 relates to a process for preparing a crosslinked hyaluronic acid gel, comprising stirring and mixing a mixture containing 10 w/v % or more of hyaluronic acid, a crosslinking agent, and water under acidic or alkaline condition.

EP 2 054 039 (WO 2008/018796) relates to a viscoelastic hydrogel composition comprising first microparticles and second microparticles capable of interacting with each other through stereocomplex interactions, wherein said first microparticles comprise a crosslinked first hydrophilic polymer, said first microparticles comprising external grafts of first oligomers or co-oligomers comprising a first chiral region, said first chiral region comprising first chiral monomers, and wherein said second microparticles comprise a crosslinked second hydrophilic polymer, said second microparticles comprising external grafts of second oligomers or co-oligomers comprising a second chiral region, said second chiral region comprising second chiral monomers, said second chiral monomers having chirality that is opposite to the chirality of said first chiral monomers, wherein said first chiral region and said second chiral region interact with each other non-covalently. The hydrophilic polymer may be hyaluronic acid.

EP 2 178 923 (WO 2009/018076) relates to a process for the preparation of crosslinked hyaluronic acid, said process comprising contacting hyaluronic acid with a polyethylene glycol-based crosslinking agent.

WO 2011/119468 relates to a hydrogel for soft tissue augmentation comprising a cross-linked biocompatible polymer having zero-length cross-linked moieties and optionally at least one other active ingredient incorporated into said cross-linked biocompatible polymer.

EP 2 152 329 (WO 2008/068297) relates to an implant that can be injected subcutaneously or intradermally in the form of a single-phase hydrogel comprising a gel composed of crosslinked hyaluronic acid and one of its physiologically acceptable salts.

EP 2 170 961 (WO 2009/021526) relates to a hyaluronic acid dispersion for use in aesthetics medicine and orthopedics, wherein the dispersed phase comprises particles made of crosslinked hyaluronic acid, and the continuous phase substantially comprises linear hyaluronic acid.

EP 1 699 500 (WO 2005/067994) relates to a hyaluronic acid composition comprising crosslinked, water-insoluble, hydrated hyaluronic acid gel particles. The composition may be used for augmenting tissue in a subject that is in need of tissue augmentation, to a method of stabilizing crosslinked HA including hydrating water-insoluble, dehydrated crosslinked HA with a physiologically compatible aqueous solution that includes a local anesthetic, wherein the value of storage modulus G' for the stabilized composition is at least about 110 % of the value of G' for a non-stabilized composition, and to the stabilized HA composition.

WO 2010/015900 relates to soft tissue fillers, for example, dermal and subdermal fillers, based on hyaluronic acids and pharmaceutically acceptable salts thereof, wherein the hyaluronic acid-based compositions may include a therapeutically effective amount of at least one anesthetic agent, for example, lidocaine. The hyaluronic acid-based compositions including lidocaine have an enhanced stability, relative to conventional compositions including lidocaine, for example when subjected to sterilization techniques or when stored for long periods of time. Methods and processes of preparing such hyaluronic acid-based compositions are also provided.

FR 2 919 999 relates to a cosmetic composition or pharmaceutical composition, which comprises a hyaluronic acid and a divalent cation. The composition may be used for treating wrinkles.

EP 2 254 584 (WO 2009/098127) relates to biocompatible injectable products capable of releasing zinc and/or at least one saccharide salt in the form of zinc, to compositions containing said products, and to the use thereof in particular for filling or increasing the volume of biological tissues or for replacing or supplementing a biological fluid.

EP 2 155 212 (WO 2008/139122) relates to the association of hyaluronic acid and at least one inhibitor of hyaluronic acid degradation, which is intended, in particular, for use in human dermatology and plastic surgery.

EP 0 839 159 B1 discloses a process for preparing a cross-linked biocompatible polysaccharide gel composition. The process comprises the cross-linking of a polysaccharide in the presence of polyfunctional cross-linking agents, wherein a viscoelastic gel is formed.

EP 1 711 552 B1 relates a method for producing a biocompatible crosslinked gel comprising steps of crosslinking a biocompatible polymer, diluting the crosslinked polymer with non-crosslinked polymer, and terminating the crosslinking reaction.

EP 0 466 300 B1 relates to a method of obtaining a biocompatible viscoelastic gel slurry, the method comprising the mixing of a biocompatible gel, which comprises crosslinked hyaluronic acid, with a second polymer, which may e.g. be hyaluronic acid, to form a two phase mixture, and to the gel as such.

It has been proposed to incorporate certain therapeutic agents, for example, anesthetic agents such as lidocaine, into injectable HA-based compositions.

WO 2013/185934 A1 discloses a method of preparing a hydrogel composition comprising crosslinked hyaluronic acid, lidocaine and/or tetracaine as an anesthetic, and optionally non-crosslinked hyaluronic acid.

US2013/203856 A1 discloses a filler composition for tissue augmentation comprising a hydrogel of hyaluronic acid cross-linked with an alkylene diamine and lidocaine.

US 8 450 475 B2 (US 2010/0028437 A1; WO 2010/015900) discloses compositions generally comprising a hyaluronic acid (HA) component crosslinked with a crosslinking agent selected from the group consisting of 1,4-butanediol diglycidyl ether (BDDE), 1,4-bis(2,3-epoxypropoxy)butane, 1,4-bisglycidyloxybutane, 1,2-bis(2,3-epoxypropoxy)ethylene and 1-(2,3-epoxypropyl)-2,3-epoxycyclohexane, and 1,4-butanediol diglycidyl ether; and at least one an anesthetic agent combined with the crosslinked HA component. The compositions are prepared by hydrating the sodium salt of hyaluronic acid (NaHA) in an alkaline solution, crosslinking NaHA, terminating the crosslinking and adding lidocain to the crosslinked product.

WO 2010/015901 relates to compositions generally comprising a hyaluronic acid (HA) component crosslinked with a crosslinking agent selected from the group consisting of 1,4-butanediol diglycidyl ether (BDDE), 1,4-bis(2,3-epoxypropoxy)butane, 1,4-bisglycidyloxybutane, 1,2-bis(2,3-epoxypropoxy)ethylene and 1-(2,3-epoxypropyl)-2,3-epoxycyclohexane, and 1,4-butanediol diglycidyl ether; and at least one an anesthetic agent combined with the crosslinked HA component. The compositions are prepared by adding lidocain to the crosslinked product.

HA-based injectable compositions are prone to partial degradation prior to injection, particularly during high temperature sterilization steps and/or when placed in storage for any significant length of time, which is associated with loss of rheological properties. Also an undesired loss of homogeneity may be observed. These drawbacks may be intensified when incorporating lidocaine during the manufacturing process.

Thus, a fundamental requirement for a minimum of side effects and safe application of hyaluronic acid - either including an anesthetic agent or not - is the provision of a gel which is as homogeneous and as degradation stable as possible.

### OBJECTS OF THE INVENTION

One object of the invention is the provision of compositions based on a polysaccharide having improved homogeneity and degradation stability, in particular compositions based on a polysaccharide such as hyaluronic acid - either including an anesthetic and/or anti-arrhythmic or not.

### SUMMARY OF THE INVENTION

The inventors of the present invention have discovered that the mode of addition of lidocain to hyaluronic acid gel and / or the pretreatment of the hyaluronic acid to be crosslinked may have a significant impact on homogeneity and degradation stability of hyaluronic acid gels.

According to a ***first aspect***, the invention relates to a method of preparing a composition comprising a crosslinked first polymer and a non-crosslinked second polymer, wherein the first and the second polymer are selected from a polysaccharide, and water, wherein the composition contains an anesthetic, comprising at least steps (S1) to (S5):
(S1) mixing the first polymer and water, wherein the pH of the mixture is in the range of from 6.5 to 7.5, and storing the mixture at a temperature of from 0 °C to 15 °C for a period of at least two hours prior to the crosslinking in step (S2);
(S2) crosslinking a mixture comprising the first polymer and water;
(S3) subsequent to the crosslinking in step (S2), terminating the crosslinking;
(S4) subjecting the product obtained after step (S3) to dialysis;
(S5) blending the product obtained after step (S4) with a mixture comprising the second polymer and water, wherein the mixture comprises an anesthetic;
   wherein the polysaccharide is a hyaluronic acid or a sodium salt thereof, and said anesthetic is lidocaine or a salt thereof.

In one embodiment, the method further comprises step (S6):
(S6) filling the product obtained after step (S5) into a syringe and sterilizing it.

According to the present invention, the polysaccharide is a hyaluronic acid or a sodium salt thereof.

In one embodiment, the first polymer has a molecular weight Mw of from 1.0 MDa to less than 3.0 MDa; and / or the second polymer has a molecular weight of at least 1.0 MDa.

In one embodiment, the first polymer comprises at least two polysaccharides having different molecular weights.

In one embodiment, the weight of the second polymer based on the weight of the first polymer is less than 5 %, or less than 4 %, e.g. is in the range of from 0.01 to 5 %, or is in the range of from 0.1 to 4 %, or is in the range of from 0.1 to 2.5 %, or from 0.2 to 2.0 %, or from 0.5 to 1.5 %.

According to the present invention the anesthetic is lidocaine, or lidocaine hydrochloride, or lidocaine hydrochloride monohydrate.

In one embodiment, said anesthetic is present in an amount of from 0.1 to 5 % by weight, based on the total amount of the composition.

According to a ***second aspect*** not according to the invention, there is described a composition, comprising a crosslinked first polymer, a non-crosslinked second polymer, wherein the first and the second polymer are selected from a polysaccharide, and water, an anesthetic, obtainable by a method as defined in the ***first aspect.***

According to a ***third aspect*** not according to the invention, there is described the use of the composition prepared according to the method as defined in the ***first aspect***, or use of the composition defined in the ***second aspect*** not according to the invention, in a cosmetic application; or use of the composition prepared according to the method as defined in the ***first aspect***, or use of the composition defined in the ***second aspect*** not according to the invention, as a dermatological filler.

### DETAILED DESCRIPTION OF THE INVENTION

According to a ***first aspect***, the invention relates to a method of preparing a composition, such as a gel, the composition comprising a crosslinked first polymer and a non-crosslinked second polymer, wherein the first and the second polymer are selected from a hyaluronic acid and/or a sodium salt thereof, and water, wherein the composition contains an anesthetic, the method comprising at least steps (S1) to (S5):
(S1) mixing the first polymer and water, wherein the pH of the mixture is in the range of from 6.5 to 7.5, and storing the mixture at a temperature of from 0 °C to 15 °C for a period of at least two hours prior to the crosslinking in step (S2);
(S2) crosslinking a mixture comprising the first polymer and water, preferably wherein the pH is above 7;
(S3) subsequent to the crosslinking in step (S2), terminating the crosslinking, preferably by adding an acid to the mixture obtained after step (S2);
(S4) subjecting the product obtained after step (S3) to dialysis;
(S5) blending the product obtained after step (S4) with a mixture comprising the second polymer and water, wherein said mixture comprises an anesthetic;
   wherein said anesthetic is lidocaine or a salt thereof.

The term "*composition*" as used in this disclosure encompasses a product comprising at least the crosslinked first polymer and water.

In one embodiment, the composition is a gel such as a hydrogel. The term "*gel*" as used herein encompasses a product, which has both viscous and elastic properties. Thus, the term encompasses a viscoelastic product. In popular science, a gel sometimes is characterized as a jelly-like material. The viscoelastic properties of a gel may be determined by determining the loss modulus and the storage modulus of the gel. The ratio between the loss module G" and the storage module G' may be expressed by the loss factor tanδ = G" / G'. The higher the loss factor, the more the properties of the product approach Newtonian flow. The viscosity of the product may be expressed in terms of η* Suitable methods for determining tanδ and η* are known in the art.

The polysaccharide is a hyaluronic acid (HA). The hyaluronic acid may be provided in the form of a salt thereof such as the sodium salt (NaHA). It is also possible to provide a mixture of the acid and a salt thereof such as the sodium salt. Thus, the term "*hyaluronic acid*" as used herein is synonymously used to terms such as "*hyaluronan*" or "*hyaluronate*"*.* HA has a well-recognized meaning in the art. It is commercially available in grades having different molecular weights (Mw) and / or different molecular weight distributions. It is available in non-crosslinked form as is used as starting material in step (S2) of the method according to the invention. It can have up to 25.000 disaccharide units in length. The molecular weight of HA may range from 5,000 to 20,000,000 Da.

### Step (S1)

In one embodiment, the method further comprises step (S1) prior to step (S2):
(S1) mixing the first polymer and water, wherein the pH of the mixture is in the range of from 6.5 to 7.5, and storing the mixture at a temperature of from 0 °C to 15 °C for a period of at least two hours prior to the crosslinking in step (S2).

Step (S1) may be termed as a "*swelling or pre-swelling step*" of the polysaccharide employed in step (S1), which is HA, prior to crosslinking according to step (S2). It may also be termed as a step in which the first polymer is treated with water.

Step (S1) according to the invention fundamentally differs from respective steps disclosed in the prior art, in which HA commonly is dissolved in alkaline solution at ambient temperature at about 23 °C as is e.g. disclosed in US 8 450 475.

The inventors of the present invention surprisingly have discovered that step (S1) may contribute to homogeneity and degradation stability of the composition prepared in the method according to the invention.

In one embodiment, the storing temperature employed in step (S1) ranges from 3 to 12 °C or from 3 to 10 °C or from 3 to 7 °C.

In another embodiment, the storing duration is at least 4 hours or at least 6 hours or at least 8 hours or at least 10 hours or at least 12 hours or at least 14 hours. In another embodiment, the storage duration is in the range of from to 24 h or from 6 to 22 h or from 8 to 20 h.

A "*pH value of from 6.5 to 7.5*" as used herein is termed as "*neutral*"*.*

### Step (S2)

Step (S2) requires the crosslinking of a mixture comprising the first polymer and water.

The term "*crosslinking*" as used herein encompasses the linking of at least two different polymer chains of the polysaccharide by means of a chemical bond or chemical bonds. As a consequence, molecular weight of the first polymer is increased, and thus viscosity and / or elasticity.

In one embodiment, crosslinking is performed via a crosslinker. Suitable crosslinkers for crosslinking polysaccharides such as hyaluronic acids are known in the art. The crosslinkers defined in the BACKGROUND section may be used. In one embodiment, a crosslinker based on an epoxide-structure may be used in the method according to the invention. In one embodiment, a diglycidyether is used for the crosslinking. In one embodiment, 1,4-butanediol diglycidylether (BDDE) is used for the crosslinking. This compound is commercially available.

In one embodiment, the crosslinker is used in a quantity of from 5 to 15 % (volume crosslinker / weight of polysaccharide such as hyaluronic acid), such as 6 to 14 % (v/w), or 7 to 12 % (v/w).

Advantageously, the temperature in the crosslinking reaction according to step (S2) is controlled. In one embodiment, crosslinking according to step (S2) is effected in a temperature range of from 0 to 40 °C. In another embodiment, the temperature in step (i) is controlled such that it proceeds in a temperature range of from 15 to 40 °C. In one embodiment, the temperature in step (S2) is from 25 to 35 °C. In one embodiment, the temperature in step (S2) is controlled such that the crosslinking proceeds in a temperature range of from 25 to 30 °C. In another embodiment, the temperature in step (S2) is controlled such that the crosslinking proceeds in a temperature range of from 30 to 35 °C, or from above 30 °C to 35 °C.

Such temperatures or temperature ranges may support the formation of homogenous crosslinking avoiding inhomogeneous particles as far as possible. Furthermore, in one embodiment, the control of the temperature allows the tailor-made adjustment of the viscoelastic properties of the composition according to the invention.

In one embodiment, if the crosslinking according to step (S2) is performed at a higher temperature, e.g. in a temperature range of from above 30 to 35 °C, the viscoelastic properties of the resulting composition such as a gel is more intense compared to composition, in which the crosslinking has been performed at a lower temperature, e.g. at a temperature of from 25 °C to 30 °C. Such differences may be characterized by determining the storage modulus and the loss modulus of the composition according to methods known in the art. Accordingly, in one embodiment, the appropriate selection of the reaction temperature in step (S2) allows for the preparation of compositions such as gels having different viscoelastic properties.

The term "*mixture*" or "*blend*" as used herein encompasses a combination of two or more substances, which are mixed but not chemically bound to one another. Thus, the term "*mixture*" or "*blend*" refers to a physical combination of the first and/ or second polymer, which is a polysaccharide, and water. The mixture may be provided in the form of a solution or a suspension or a colloid.

The first polymer, i.e. the first polysaccharide, which is HA, in general has a molecular weight Mw in the range of from 1.0 to 4.0 MDa, or from 1.5 MDa to less than 3.5 MDa, or from 2.0 MDa to less than 3.5 MDa. These ranges of molecular weight Mw relate to ranges before crosslinking, respectively.

In one embodiment, the first polysaccharide has a molecular weight Mw of from 2.0 MDa to less than 3.0 MDa, in turn before crosslinking.

In one embodiment, the first polymer includes two polymers P1 and P2 having different molecular weights. In one embodiment, P1 has a molecular weight in the range of from 1 to 2 MDa, and P2 has a higher molecular weight compared thereto, which is in the range of from 2 to 3 MDa.

In one embodiment, the weight ratio of P1/P2 is in the range of from 9 : 1 to 6 : 4, or from 7 : 1 to 6 : 4, or from 6 : 1 to 4 : 1

Employing two polymers P1 and P2 as first polymer may further support homogeneity and degradation stability.

Water may be provided in the form of pipe water, distilled water, or deionized water.

In one embodiment, the mixture used in step (S2) additionally comprises a buffer solution.

In one embodiment, the buffer solution is a phosphate buffer solution. In one embodiment, said phosphate buffer solution is made from sodium chloride, dibasic anhydrous sodium phosphate, monobasic sodium phosphate dihydrate and water. In one embodiment, the buffer solution comprises mannitol.

In one embodiment, the buffer is an alkaline buffer.

In one embodiment, the pH of the buffer is from 6.8 to 7.6, or from 7.0 to 7.4, or from 7.1 to 7.3.

In one embodiment, step (S2) may be effected by mixing the first polymer, water, the crosslinker, and optionally buffer solution, and stirring the mixture for a predetermined time, wherein the temperature is controlled to not exceed the predetermined setpoint.

### Step (S3)

Step (S3) requires the termination of the crosslinking effected in step (S2).

The termination of the crosslinking reaction according to step (S2) is mandatorily necessary since otherwise composition or gels may be obtained having a viscosity or a viscosity and elasticity being too high to allow for the appropriate use as dermatological filler, respectively the viscosity or the viscoelastic properties of the composition or gel are not constant as long as the gel contains compounds that may affect crosslinking such as the crosslinker used in step (S2).

Basically, each compound capable of reacting with the crosslinker and thus deactivating it may be used for terminating the crosslinking reaction.

Since, in one embodiment, crosslinkers of the epoxide type are used in step (i), termination of the crosslinking may be effected by means of the addition of compounds, which cleave the epoxide moiety such that no further crosslinking with suitable groups in the polysaccharide can occur. In one embodiment, cleavage of the epoxide and thus termination of the crosslinking in step (i) may be effected by an acid. Organic acids as well as inorganic acids may be used for terminating crosslinking. In one embodiment, an inorganic acid such as hydrochloric acid is used.

The compound used for termination of the crosslinking reaction may be applied in a buffer solution, e.g. in the buffer solution as used in step (S2). Such solution may be termed as "*quench solution*"*.* Accordingly, termination according to step (S3) may be effected by quenching the crosslinked mixture obtained according to step (S2).

In one embodiment, the termination of crosslinking according to step (S3) is effected in a temperature range of from 0 °C to 30 °C. In another embodiment, the temperature in step (S3) is controlled such that it does not exceed a temperature of 20 °C, or 15 °C. In one embodiment, the temperature in step (S3) is from 0 to 10 °C, or from 3 to 7 °C, e.g. 5 °C.

The termination according to step (S3) may be effected by adding the compound used for termination to the mixture according to step (S2), and e.g. stirring it for a predetermined time.

The termination of the crosslinking reaction is crucial for the method according to the invention, since the thus obtained composition or gel has an excellent stability, i.e. it does not change its properties, in particular its viscosity or its viscoelastic properties, after application, e.g. after use as dermatological filler in the skin tissue.

### Step (S4)

Step (S4) requires the subjection of the product obtained in step (S3) to dialysis.

In one embodiment, this step serves for the removal of extraneous compounds or particles from the gel obtained in step (S3). Extraneous compounds or particles might negatively affect the physical properties of the composition such as a gel and / or might adversely affect the compatibility of the composition or gel with skin tissue. Thus, in one embodiment, step (S4) serves for the lowering or for the avoidance of possible inflammatory reactions when the composition according to the invention is injected into skin tissue.

In another embodiment, this step serves for adjusting the swelling of the gel obtained in step (S3). The term "*swelling*" or "*swellability*" as used herein encompasses the water take-up of the gel, respectively the water take-up of hyaluronic acid.

In one embodiment, the swelling of the gel obtained in step (S3) when subjected to step (S4), i.e. the water-take up during dialysis, is from 5 to 25 % based on the total weight of the gel such as from 6 to 23 %, or from 7 to 22 %, or from 9 to 21 %. In another embodiment, the swelling or swellability is from 7 to 18 %, or from 8 to 15 %.

In one embodiment, such swelling or swellability creates a swelling pressure that enables the HA matrix to withstand compressive forces, e.g. when injected into skin tissue and said skin tissue is exposed to compressive force.

In one embodiment, step (S4) serves for the removal of extraneous compounds or particles from the gel obtained in step (S3) and for adjusting the swelling of the gel obtained in step (S3).

Thus, step (S4) serves, among others, for the provision of a further improved HA composition as compared to products known in the art.

Thus, besides step (S3) (termination of the crosslinking), step (S4) (dialysis) is a further crucial reaction step in the sequence of steps required for the preparation of the composition such according to the invention. In particular the combination of step (S3) and step (S4) in the reaction sequence according to the invention allows the provision of a composition such as a gel having the properties, which should be achieved according to the posed problem.

In one embodiment, dialysis is performed using a dialysis membrane having a predetermined molecular weight cut off. Such dialysis membranes are commercially available. The term "*molecular weight cut off (MWCO)*" as used herein refers to the lowest molecular weight solute (in Daltons) in which a defined percentage of the solute is retained by the membrane used for dialysis, or refers to the molecular weight at which a defined percentage of the analytes are prohibited from membrane diffusion. Commercially available dialysis membranes typically have MWCOs that range from 1,000 to 100,000 Da. In one embodiment, the used dialysis membrane has a MWCO in the range of from 12,000 to 14,000 Da.

In one embodiment, dialysis is performed using a dialysis solution comprising a buffer. In one embodiment, the buffer is the buffer used in step (S2), or used in step (S3).

In one embodiment, the dialysis according to step (S4) is effected in a temperature range of from 0 °C to 50 °C or from 0 °C to 30 °C. In another embodiment, the temperature in step (S4) is controlled such that it does not exceed a temperature of 20 °C, or 15 °C. In one embodiment, the temperature in step (S4) is from 0 to 10 °C, or from 3 to 7 °C, e.g. 5 °C.

In one embodiment, the method according to the invention is performed such that the temperature in steps (S2) to (S4) is from 0 to 10 °C, respectively; or the method according to the invention is performed such that the temperature in steps (S2) to (S4) is from 3 to 7 °C, e.g. 5 °C, respectively.

In one embodiment, dialysis is performed by stirring the content of the container. In one embodiment, the dialysis solution may be exchanged once or at least twice by a fresh dialysis solution. In one embodiment, the interval of exchange ranges from 8 to 18 hours or from 10 to 14 hours, such as 12 ± 2 hours. In one embodiment, dialysis according to step (S3) is allowed to proceed for 30 to 45 hours or from 35 to 39 hours, such as 37 ± 2 hours.

In one embodiment, prior to subjecting the product obtained in step (S3) to dialysis, or after the product has been subjected to dialysis according to step (S4), the product may be subjected to a sieving step, or several sieving steps, in order to further homogenize the product, respectively to remove inhomogeneous particles or any further particles, which might negatively affect the use as dermatological filler.

The term "*sieving step*" encompasses a step in which a product obtained in any one of steps (S3) to (S4) is extruded through a sieve. The term "*extruded through* a *sieve*" encompasses terms such as "*passed through a sieve*", or "*pressed through* a *sieve*" or "*directed through* a *sieve*" or "*filtered*"*.*

The term "sieve" encompasses the term "*filter*"*.* The term "*sieve*" or "*filter*" encompasses any device having pores or holes through which a liquid may penetrate, wherein particles, which may be contained in the liquid, may be removed or which may be sheared to fit through the pores of the sieve or the filter. Thus, in one embodiment, the particles are resized by sieving or filtering. The sieve may e.g. be provided in the form of a net of metal wires or fibers such as plastic fibers. Suitable sieves are known in the field of sieving and filtering.

Thus, in one embodiment, step (S3) and / or step (S4) further comprise(s) at least step (S3.1) and / or step (S4.1):
(S3.1) extruding the product obtained in step (S3) through a sieve;
(S4.1) extruding the product obtained in step (S4) through a sieve.

In one embodiment, tanδ of the composition obtained in step (S4) ranges from 0.1 to 0.9 measured at a frequency of 0.7 Hz and 30 °C such as from 0.1 to 0.5, or from 0.2 to 0.4. In another embodiment, tanδ of the composition obtained in step (S4) ranges from 0.1 to 3.5 measured at a frequency of 0.7 Hz and 30 °C. In one embodiment, η* is in the range of from 2,000 mPa*s to 200,000 mPa*s at a frequency of 0.7 Hz and 30 °C, wherein tanδ ranges from 0.1 to 0.9, such as from 0.1 to 0.5, or from 0.2 to 0.4. In one embodiment, tanδ of the composition obtained in step (S4) ranges from 0.10 to 3.5 measured at a frequency of 0.7 Hz and 30 °C. In one embodiment, corresponding viscosity η* is in the range of from 2,500 mPa*s to 145,000 mPa*s, or from 4,000 to 145,000 mPa*s. In one embodiment, tanδ of the composition obtained in step (S4) ranges from 0.10 to 3.5 measured at a frequency of 0.7 Hz and 30 °C. In one embodiment, tanδ of the composition obtained in step (S4) ranges from 0.10 to 0.25 measured at a frequency of 0.7 Hz and 30 °C.

### Step (S5)

Step (S5) requires the blending of the product obtained in step (S4) with the second polymer and water, wherein the second polymer and water comprise an anesthetic.

The term "*second polymer and water*" encompasses a mixture comprising the second polymer and water.

Although the composition according to the invention allows for an use, which avoids adverse skin reaction as far as possible, a local anesthetic drug may be added to the composition or gel according to the invention. Such drug may relieve itching, burning and pain, which might arise from skin inflammation when the composition or gel according to the invention is injected into skin tissue.

According to the present invention, lidocaine is used as a local anesthetic drug. This drug is known for e.g. injection as a dental anesthetic or as a local anesthetic for minor surgery. In one embodiment, lidocaine is used in the form of a salt such as the hydrochloride and / or in the form of a hydrate such as the monohydrate. Accordingly, the term "*lidocaine*" as used herein, encompasses the salts and hydrates thereof.

In one embodiment, lidocaine is used in an amount of from 0 to 1 % by weight based on the weight of the composition or gel, or from 0 to 0.5 wt.-%. In one embodiment, the weight is from 0.3 % to 0.35 %. In one embodiment, the weight is 0.3 % or is 0.35 %.

In one embodiment, the method according to the invention further comprises admixing lidocaine, or lidocaine hydrochloride, or lidocaine hydrochloride monohydrate to the second polymer and water.

The term "*blending*" as used herein encompasses the mixing of the crosslinked polymer obtained in step (S4) with the second polymer and water comprising an anesthetic and/or anti-arrhythmic.

The second polymer is a polysaccharidethat is also HA. This polymer may be the same polymer as the first polymer, or may be different therefrom. Thus, in one embodiment, the second polymer is the same HA as the first polymer used in step (S2), or is a HA that is different from the HA used in step (S2).

In one embodiment, the first polymer has the same molecular weight as the second polymer. In another embodiment, the molecular weights are different from one another. The term "*molecular weight*" of said second polymer refers to the respective molecular weight of said polymer before blending and optionally crosslinking said second polymer.

In one embodiment, the second polymer has a molecular weight of at least 1.0 MDa, or of at least 2.0 MDa, or of at least 3.0 MDa, or of at least 3.5 MDa, or of at least 4.0 MDa. In one embodiment, the second polymer has a molecular weight of at least 1.0 MDa, wherein the upper limit is 20 MDa, or 10 MDa, or 8 MDa, or 6 MDa, or 4 MDa, or 3.0 MDa, or 2.0 MDa, respectively. In one embodiment, the second polymer has a molecular weight of at least 2.0 MDa, or 3.0 MDa, or 3.5 MDa, wherein the upper limit is 20 MDa, or 10 MDa, or 8 MDa, or 6 MDa, or 4 MDa, respectively. In one embodiment, the second polymer has a molecular weight of at least 4.0 MDa, wherein the upper limit is 20 MDa, or 10 MDa, or 8 MDa, or 6 MDa, respectively.

In one embodiment, the second polymer is provided in a buffer such as the buffer, which may be used in step (S2) or in step (S3) or step (S4).

In one embodiment, the blending according to step (S5) is effected in a temperature range of from 0 °C to 40 °C or from 0 °C to 30 °C. In another embodiment, the temperature in step (S5) is controlled such that it does not exceed a temperature of 20 °C or 15 °C. In one embodiment, the temperature in step (S5) is from 0 to 10 °C or from 3 to 7 °C; e.g. 5 °C.

In one embodiment, blending according to step (S5) is performed by stirring the crosslinked product, wherein the crosslinking has been terminated according to step (S3), with the second polymer comprising an anesthetic.

In one embodiment, the weight of the second polymer based on the weight of the first polymer is less than 5 %, or less than 4 %, e.g. is in the range of from 0.01 to 5 %, or is in the range of from 0.1 to 4 %, or is in the range of from 0.1 to 2.5 %, or from 0.2 to 2.0 %, or from 0.5 to 1.5 %.

In one embodiment, the pH of the final composition prepared according to the method of the invention is adjusted to a range of from 6.5 to 7.5 such as 6.7 to 7.2, or 6.8 to 7.1 or 6.8 to 6.9. Such pH may support the compatibility of the composition with skin tissue. For the adjustment of said pH, in one embodiment, said buffer used in step (S2) may be used.

In one embodiment, step (S5) allows the tailor-made adjustment of properties with respect to viscosity and elasticity, or viscosity or elasticity of the target composition or gel, which are essential for the use as dermatological filler. Such properties may be achieved by selecting a suitable temperature range such in step (S2) and / or weight ratio between the first and the second polymer in step (S5) and / or quantity of crosslinker used in step (S3).

In one embodiment, tanδ of the composition obtained in step (S5) ranges from 0.1 to 0.9 measured at a frequency of 0.7 Hz and 30 °C such as from 0.1 to 0.5, or from 0.2 to 0.4. In another embodiment, tanδ of the composition obtained in step (S5) ranges from 0.1 to 3.5 or from 0.15 to 3.4 measured at a frequency of 0.7 Hz and 30 °C. In one embodiment, η* is in the range of from 2,000 mPa*s to 200,000 mPa*s at a frequency of 0.7 Hz and 30 °C, wherein tanδ ranges from 0.1 to 0.9, such as from 0.1 to 0.5, or from 0.2 to 0.4. In one embodiment, tanδ of the composition obtained in step (S5) ranges from 0.10 to 3.5 measured at a frequency of 0.7 Hz and 30 °C. In one embodiment, corresponding viscosity η* is in the range of from 2,500 mPa*s to 145,000 mPa*s, or from 4,000 to 145,000 mPa*s. In one embodiment, tanδ of the composition obtained in step (S5) ranges from 0.10 to 3.5 measured at a frequency of 0.7 Hz and 30 °C. In one embodiment, tanδ of the composition obtained in step (S5) ranges from 0.10 to 0.25 measured at a frequency of 0.7 Hz and 30 °C. In one embodiment, tanδ of the composition obtained in step (S5) ranges from 0.1 to 3.5 measured at a frequency of 0.7 Hz and 30 °C. In one embodiment, corresponding viscosity η* is in the range of from 2,000 mPa*s to 150,000 mPa*s. In one embodiment, tanδ of the composition obtained in step (S5) ranges from 0.15 to 3.5 measured at a frequency of 0.7 Hz and 30 °C. In one embodiment, corresponding viscosity η* is in the range of from 2,500 mPa*s to 145,000 mPa*s, or from 4,000 to 145,000 mPa*s. In one embodiment, tanδ of the composition obtained in step (v) ranges from 0.15 to 0.25 measured at a frequency of 0.7 Hz and 30 °C. In one embodiment, the corresponding viscosity η* is in the range of from 15,000 mPa*s to 28,000 mPa*s.

In one embodiment, the method according to the invention further comprises step (S5.0) prior to step (S5):
(S5.0) admixing an anesthetic or anti-arrhythmic or an anesthetic and anti-arrhythmic to the second polymer and water.

### Step (S6)

Finally, in one embodiment, the product obtained in step (S5) may be filled into a syringe. This is since the product obtained in the method according to the invention is intended to be injected for application. In one embodiment, the product obtained in step (S5) is filled into a syringe, and is sterilized. In one embodiment, the product obtained in step (S5) is extruded into the syringe, whereby the filling is effected.

Accordingly, in one embodiment, the method according to the invention further comprises step (S6) after step (S5):
(S6) filling the product obtained in step (S5) into a syringe and sterilizing it.

Sterilization may be effected by methods known in the art. The term "*sterilization*" as used herein encompasses any process that eliminates or removes or kills all forms of microbial life, including transmissible agents (such as fungi, bacteria, viruses, spore forms, etc.) present on the surface of the syringe and / or in the composition or gel prepared according to the method of the invention. Sterilization may be achieved by the methods known in the art such as applying heat, chemicals, irradiation, high pressure or filtration, or a proper combination thereof.

In one embodiment, sterilization is effected prior to the filling according to step (S6), i.e. the composition or gel obtained in step (S5) and the syringe are sterilized independently from one another. In another embodiment, sterilization is effected during the filling according to step (S6). In another embodiment, sterilization is effected after the filling according to step (S6).

According to a ***second aspect*** not according to the invention, there is described a composition, such as a gel, the composition comprising a crosslinked first polymer, a second non-crosslinked polymer, and water, wherein the first and the second polymer are selected from a polysaccharide that is hyaluronic acid and/or a sodium salt thereof, an anesthetic, obtainable by a method as defined in the ***first aspect.***

According to a ***third aspect*** not according to the invention, there is described the use of the composition prepared according to the method as defined in the ***first aspect,*** or use of the composition as defined in the ***second aspect aspect*** not according to the invention, in a cosmetic application; or use of the composition prepared according to the method as defined in the ***first aspect***, or use of the composition as defined in the ***second aspect*** not according to the invention, as a dermatological filler.

The term "*dermatological filler*" as used herein means that the composition such as a gel prepared according to the method of the invention is suitable for increasing the volume of skin tissue, i.e. to augment skin tissue.

In one embodiment, the composition such as a gel is used to augment skin tissue such as to augment skin face tissue, and / or to correct moderate or deep wrinkles.

In one embodiment, the composition is an injectable composition, i.e. it is injected into skin tissue when applied.

In one embodiment, the overall content of polysaccharide, which is HA, in the final composition as defined in the ***second aspect*** not according to the invention is in the range of from 1 to 5 % by weight based on the total weight of the composition. In another embodiment, the overall content is in the range of from 1.5 to 4 % by weight, or from 2 to 2.5 % by weight.

In one embodiment, the product obtained according to the method of the invention is an isotonic, sterile, viscoelastic composition such as a gel. This composition or gel is injectable and may act as an implant to increase the volume of skin tissue, i.e. to augment it. In one embodiment, the product is an isotonic, sterile, viscoelastic injectable gel or implant to increase the volume of e.g. face skin tissue, or to correct moderate or deep wrinkles.

In one embodiment, the skin tissue comprises or is the tissue of the lips. In other embodiments, the skin tissue comprises or is the skin tissue of moderate to severe facial wrinkles or folds, such as nasolabial folds.

In one embodiment, the composition prepared according to the ***first aspect*** of the invention, provides for a safe and effective, biocompatible, non-immunogenic composition, which is easy to distribute and store, and which should not require allergy testing. Additionally, the composition has an acceptable persistency when applied to skin tissue. In one embodiment, the composition prepared according to the method of the invention is stable for a considerable time period, when applied to skin tissue.

Accordingly, in one embodiment, the invention relates to a method of preparing a composition according to the ***first aspect***, wherein the composition obtained in step (S5) is dialyzed in step (S4) such as to have a pH in the range of from 6.5 to 7.5, such as 6.7 to 7.2, or from 6.8 to 7.1 or from 6.8 to 6.9.

In one embodiment, the invention relates to a method of preparing a composition according to the ***first aspect,*** wherein the gel obtained in step (S3) is dialyzed in step (S4) such as to have a swellability in the range of from 5 to 25 % based on the total weight of the gel, such as from 6 to 23 % or from 7 to 22 % or from 9 to 21 %.

In one embodiment, the invention relates to a method of preparing a composition according to the ***first aspect***, wherein the composition, when injected into skin tissue, is stable for at least three months, such as for at least 4 months, or five months, or six months.

In one embodiment, the invention relates to a method of preparing a composition according to the ***first aspect***, wherein said anesthetic, which is lidocaine, of step (v), is released when injected into skin tissue; and wherein the composition is sterile.

In one embodiment, the invention relates to a method of preparing a composition according to the ***first aspect***, wherein the overall content of HA in the final composition, such as a gel, is in the range of from 1 to 5 % by weight based on the total weight of the composition, such as from 1.5 to 4 % by weight, or from 2 to 2.5 % by weight.

In one embodiment, the invention relates to a method of preparing a composition according to the ***first aspect***, wherein the composition does not irritate skin tissue when injected into said skin tissue.

In one embodiment, the invention relates to a method of preparing a composition according to the ***first aspect***, wherein the composition is used as an injectable tissue filler while the composition is in the form of a gel.

In one embodiment, the invention relates to a method of preparing a composition according to the ***first aspect***, wherein the hyaluronic acid, or the hyaluronic acid and the anesthetic, are the only active ingredients of the composition.

In one embodiment, the invention relates to a method of preparing a composition according to the ***first aspect***, wherein loss factor tanδ = G" / G' of the product obtained in step (S4) is not increased by 10 % or 15 % or 20 % or 25 % compared to a product obtained in step (S6).

### EXAMPLES

### Example 1 (not according to the invention)

### Preparation of buffer solution

A buffer solution is made from sodium chloride, dibasic anhydrous sodium phosphate, monobasic sodium phosphate dihydrate and water by dissolving the salts in water.

### Preparation of HA in buffer

Subsequent to the preparation of the buffer solution, sodium hyaluronate having a molecular weight of from 2.5 MDa to less than 3.0 MDa and sodium hyaluronate having a molecular weight of from 1.0 MDa to less than 2.0 MDa are added to a quart mixing bowl and a portion of the buffer solution is added to it. The contents are mixed for 2.0 to 2.5 hours using a stirrer at 250 rpm while the jacket setpoint on the mixing bowl is set to 50 °C. Subsequent to the mixing, the contents are then cooled to 5 to 7 °C.

### Addition of alkaline solution

A first alkaline solution is prepared by dissolving sodium hydroxide in the above buffer solution. Then, a second alkaline solution is prepared by dissolving sodium hydroxide in the above buffer solution. The first alkaline solution is then added to the contents in the mixing bowl and the contents are mixed for 30 to 40 minutes at 250 rpm at a 5 °C jacket set point.

### Crosslinking reaction [step (S2)]

The crosslinking solution is prepared by adding BDDE to a portion of the second alkaline buffer solution. This alkaline solution comprising the crosslinking agent BDDE is added to the contents in the mixing bowl and allowed to mix for 10 to 15 minutes at 500 rpm at a 5 °C jacket setpoint. The mixing speed is then lowered to 100 rpm and the temperature set point is changed to a temperature of 30 °C. After a temperature of 28 °C has been reached, the mixing is turned off, and the contents are allowed to set for approx. 3 hours.

### Preparation of a quench solution for the termination of the crosslinking reaction

A 1 M HCl solution is added to a portion of the buffer solution to create a quench solution.

### Quenching reaction [step (S3)]

The temperature setpoint on the jacket is set to 5 °C and the quench solution is added to the bowl contents. The contents are then mixed for 10 to 15 minutes at 500 rpm.

### Processing of the crosslinked HA

The polymer resulting from step (S3) is then cut into pieces, which may be formed as chunks or strips. The size of the chunks or stripes may be 1,27 cm x 1,27 cm x 1,27 cm (0.5 x 0.5 x 0.5 inches) or smaller. The chunks or strips are then mixed for approximately 2.5 to 3.0 hours at 150 rpm at a 5 °C jacket setpoint. Subsequent to the mixing, the mixed product is extruded through a 558.8 µm µm [0.022 ") screen and placed back into the mixing bowl and further mixed for 2.0 to 2.5 hours at 150 rpm at a 5 °C jacket setpoint.

### Dialysis reaction [step (S4)]

Dialysis membranes having a MWCO of from 12,000 to 14.000 Da are hydrated in sterile water. Then, the gel obtained in step (S3) is extruded through a screen having a mesh size of approximately 380 µm, such as 381 µm (0.015"), then through a screen having a mesh size of approximately 200 µm, such as 203.2 µm (0.008"), then through a screen having a mesh size of approximately 140 µm, such as 139.7 µm (0.0055"), into the dialysis membranes. The dialysis membranes are filled and have an effective length of approximately 20.3 cm (8 inches) and an overall length of approximately 25.4 cm (10 inches). The membranes are then placed into a container containing the above buffer solution. The container is cooled down to 5 °C setpoint and the content is stirred. The dialysis solution is exchanged twice at an interval of 12 ± 2 hours. The dialysis is allowed to proceed for 37 ± 2 hours.

### Provision of the second polymer and lidocain

HA (sodium salt) having Mw ≥ 3.0 MDa and lidocaine HCl monohydrate are added to a portion of the buffer solution, wherein the manount of lidocain HCL monohydrate corresponds to an amount of 0.35 wt.-% based on gel. The contents are mixed with an overhead mixer for a short period of time.

### Addition of the mixture containing the second polymer and lidocain to crosslinked HA according to step (S5)

The mixture comprising the second polymer and lidocain is added to the purified product obtained in step (S4) while stirring.

### Filling the final composition into a syringe according to step (S6)

The resulting material from step (S5) is extruded into syringes and is steam sterilized.

The product obtained after step (S5) and contained in the syringe after step (S6) is an isotonic, sterile, viscoelastic injectable gel. This gel may be used as an implant suitable to increase the volume of e.g. face skin tissue, i.e. to augment said skin tissue, and / or to correct moderate or deep wrinkles of the skin.

### Example 2 (according to the invention)

The reaction is performed according to Example 1 with the difference that additionally step (S1) is performed. Sodium hyaluronate having a molecular weight of from 2.5 MDa to less than 3.0 MDa and sodium hyaluronate having a molecular weight of from 1.0 MDa to less than 2.0 MDa are added to water and the pH is adjusted to a range of from 6.5 to 7.5 using buffer, if necessary. The mixture is stirred and kept at a temperature of from 5 to 7 °C for a time period of from 10 to 12 h. The stored mixture is subjected to crosslinking and is further processed as described in Example 1.

The product obtained after step (S5) and contained in the syringe after step (S6) is an isotonic, sterile, viscoelastic injectable gel. This gel may be used as an implant suitable to increase the volume of e.g. face skin tissue, i.e. to augment said skin tissue, and / or to correct moderate or deep wrinkles of the skin.

## Claims

1. A method of preparing a composition comprising a crosslinked first polymer and a non-crosslinked second polymer, wherein the first and the second polymer are selected from a polysaccharide, and water, wherein the composition contains an anesthetic, comprising at least steps (S1) to (S5):
(S1) mixing the first polymer and water, wherein the pH of the mixture is in the range of from 6.5 to 7.5, and storing the mixture at a temperature of from 0 °C to 15 °C for a period of at least two hours prior to the crosslinking in step (S2);
(S2) crosslinking a mixture comprising the first polymer and water;
(S3) subsequent to the crosslinking in step (S2), terminating the crosslinking;
(S4) subjecting the product obtained after step (S3) to dialysis;
(S5) blending the product obtained after step (S4) with a mixture comprising the second polymer and water, wherein the mixture comprises an anesthetic;
wherein the polysaccharide is a hyaluronic acid or a sodium salt thereof, and said anesthetic is lidocaine or a salt thereof.

2. The method of claim 1, further comprising step (S6):
(S6) filling the product obtained after step (S5) into a syringe and sterilizing it.

3. The method of claim 1 or 2, wherein the first polymer has a molecular weight Mw of from 1.0 MDa to less than 3.0 MDa; and/or the second polymer has a molecular weight of at least 1.0 MDa.

4. The method of claim 3, wherein the first polymer comprises at least two polysaccharides having different molecular weights.

5. The method of any one of the preceding claims, wherein the weight of the second polymer based on the weight of the first polymer is less than 5 %, or less than 4 %, or is in the range of from 0.01 to 5 % or from 0.1 to 4 %, or is in the range of from 0.1 to 2.5 % or from 0.2 to 2.0 % or from 0.5 to 1.5 %.

6. The method of any one of the preceding claims, wherein said anesthetic is present in an amount of from 0.1 to 5 % by weight, based on the total amount of the composition.

## Patentansprüche

1. Verfahren zur Herstellung einer Zusammensetzung, umfassend ein vernetztes erstes Polymer und ein unvernetztes zweites Polymer, wobei das erste und das zweite Polymer ausgewählt ist aus einem Polysaccharid, und Wasser, wobei die Zusammensetzung ein Anästhetikum enthält und mindestens die Schritte (S1) bis (S5) umfasst:
(S1) Mischen des ersten Polymers und Wasser, wobei der pH-Wert der Mischung im Bereich von 6,5 bis 7,5 liegt, und Lagern der Mischung bei einer Temperatur von 0 °C bis 15 °C für einen Zeitraum von mindestens zwei Stunden vor dem Vernetzen in Schritt (S2);
(S2) Vernetzen einer Mischung, die das erste Polymer und Wasser umfasst;
(S3) nach dem Vernetzen in Schritt (S2), Beenden des Vernetzens;
(S4) Aussetzen des nach Schritt (S3) erhaltenen Produkts einer Dialyse;
(S5) Mischen des nach Schritt (S4) erhaltenen Produkts mit einer Mischung, die das zweite Polymer und Wasser umfasst, wobei die Mischung ein Anästhetikum umfasst;
wobei das Polysaccharid eine Hyaluronsäure oder ein Natriumsalz davon ist und das Anästhetikum Lidocain oder ein Salz davon ist.

2. Verfahren nach Anspruch 1, ferner umfassend Schritt (S6):
(S6) Füllen des nach Schritt (S5) erhaltenen Produkts in eine Spritze und Sterilisieren derselben.

3. Verfahren nach Anspruch 1 oder 2, wobei das erste Polymer ein Molekulargewicht Mw von 1,0 MDa bis weniger als 3,0 MDa aufweist; und/oder das zweite Polymer ein Molekulargewicht von mindestens 1,0 MDa aufweist.

4. Verfahren nach Anspruch 3, wobei das erste Polymer mindestens zwei Polysaccharide mit unterschiedlichen Molekulargewichten umfasst.

5. Verfahren nach einem der vorhergehenden Ansprüche, wobei das Gewicht des zweiten Polymers, bezogen auf das Gewicht des ersten Polymers, weniger als 5% oder weniger als 4% beträgt oder im Bereich von 0,01 bis 5% oder von 0,1 bis 4% liegt oder im Bereich von 0,1 bis 2,5% oder von 0,2 bis 2,0% oder von 0,5 bis 1,5% liegt.

6. Verfahren nach einem der vorhergehenden Ansprüche, wobei das Anästhetikum in einer Menge von 0,1 bis 5 Gew.-% vorliegt, bezogen auf die Gesamtmenge der Zusammensetzung.

## Revendications

1. Procédé de préparation d'une composition comprenant un premier polymère réticulé et un deuxième polymère non réticulé, dans lequel les premier et deuxième polymères sont choisis parmi les polysaccharides, et de l'eau, dans lequel la composition contient un anesthésiant, comprenant au moins les étapes (S1) à (S5) :
(S1) mélange du premier polymère et d'eau, dans lequel le pH du mélange est situé dans la plage allant de 6,5 à 7,5, et stockage du mélange à une température de 0°C à 15°C pendant une période d'au moins deux heures avant la réticulation dans l'étape (S2) ;
(S2) réticulation d'un mélange comprenant le premier polymère et de l'eau ;
(S3) suite à la réticulation dans l'étape (S2), achèvement de la réticulation ;
(S4) soumission à une dialyse du produit obtenu après l'étape (S3) ;
(S5) combinaison du produit obtenu après l'étape (S4) avec un mélange comprenant le deuxième polymère et de l'eau, dans laquelle le mélange comprend un anesthésiant ;
dans lequel le polysaccharide est un acide hyaluronique ou un sel sodique de celui-ci, et ledit anesthésiant est la lidocaïne ou un sel de celle-ci.

2. Procédé selon la revendication 1, comprenant en outre l'étape (S6) :
(S6) remplissage d'une seringue avec le produit obtenu après l'étape (S5) et stérilisation.

3. Procédé selon la revendication 1 ou 2, dans lequel le premier polymère a une masse moléculaire Mw de 1,0 MDa à moins de 3,0 MDa ; et/ou le deuxième polymère a une masse moléculaire d'au moins 1,0 MDa.

4. Procédé selon la revendication 3, dans lequel le premier polymère comprend au moins deux polysaccharides ayant des masses moléculaires différentes.

5. Procédé selon l'une quelconque des revendications précédentes, dans lequel le poids du deuxième polymère par rapport au poids du premier polymère est inférieur à 5 %, ou inférieur à 4 %, ou est situé dans la plage allant de 0,01 à 5 % ou de 0,1 à 4 %, ou est situé dans la plage allant de 0,1 à 2,5 % ou de 0,2 à 2,0 % ou de 0,5 à 1,5 %.

6. Procédé selon l'une quelconque des revendications précédentes, dans lequel ledit anesthésiant est présent en une quantité de 0,1 à 5 % en poids par rapport à la quantité totale de la composition.
